# EUROPEAN PATENT SPECIFICATION

(11) **EP 2 815 747 B1**
(45) Date of publication and mention of the grant of the patent: **02.05.2018**
(21) Application number: 12868824.9
(22) Date of filing: 12.12.2012
(51) Int. Cl.: A61K 47/10, A61K 47/20, A61K 9/70, A61K 31/4525, A61K 31/445, A61K 31/4725

(54) **INTRAORALLY DISSOLVING FILM PREPARATION**
LÖSLICHES ORALES FILMPRÄPARAT
PRÉPARATION DE FILM BUCCAL SOLUBLE

(30) Priority: 14.02.2012 JP 2012029808
(43) Date of publication of application: 24.12.2014
(73) Proprietor: Kyukyu Pharmaceutical Co., Ltd., Tokyo 103-0023 (JP)
(72) Inventor: ISHISE, Akihiro, Imizu-shi Toyama 939-0351 (JP); NISHIKAWA, Hisanobu, Imizu-shi Toyama 939-0351 (JP)
(74) Representative: Blodig, Wolfgang
(86) International application number: PCT/JP2012/082210
(87) International publication number: WO 2013/121663

(56) References cited:
- WO-A1-2008/149440
- WO-A1-2011/020610
- JP-A- 2006 519 202
- JP-A- 2010 270 142
- JP-A- 2012 240 949
- DATABASE WPI Week 201115 Thomson Scientific, London, GB; AN 2011-B06406 XP002741904, -& JP 2011 016781 A (TEIKA SEIYAKU KK) 27 January 2011 (2011-01-27)

## Description

### Technical Field

The present invention relates to an intraoral soluble-type film preparation in which a bitter taste and/or an astringent taste derived from a drug is masked.

### Background Art

Of preparations for oral administration, a film preparation, in particular, an intraoral soluble-type film preparation has been researched, developed and widely used in recent years because the preparation has advantages in, for example, that the preparation can be taken without water, that the preparation can be taken by even a person who has lowered ability to swallow, such as an elderly person and that the preparation is excellent in portability (Patent Literatures 1 to 3). Patent literature 4 discloses a film composition comprising sodium lauryl sulfate and macrogol.

### Citation List

### Patent Literature

[Patent Literature 1] JP-A-11-116469
[Patent Literature 2] JP-A-2004-43450
[Patent Literature 3] JP-A-2005-263704
[Patent Literature 4] JP-A-2011-016781

### Summary of Invention

### Technical Problem

The film preparation has a feature in that the film preparation is thin and has a wider area per unit weight, as compared to an orally disintegrating tablet. That is, the film preparation has a feature in that when the film preparation is taken, the film preparation is dissolved in the oral cavity and spreads to a wider area, as compared to the orally disintegrating tablet or the like. Because of the feature, when a drug having an unpleasant taste such as a bitter taste is blended in the film preparation, the film preparation tends to cause a further stronger unpleasant taste, as compared to the conventional orally disintegrating tablet or the like.

Therefore, an object of the present invention is to provide an intraoral soluble-type film preparation in which an unpleasant taste of a drug is masked.

### Solution to Problem

In view of the foregoing, the inventors of the present invention have conducted various researches about a means for masking an unpleasant taste of an intraoral soluble-type film preparation containing a drug having an unpleasant taste such as a bitter taste or an astringent taste in a drug layer, and as a result, have found that an intraoral soluble-type film preparation in which the bitter taste and/or astringent taste of the drug is significantly masked can be achieved by blending an alkyl sulfate at a specific molar ratio with respect to the drug. Thus, the inventors have accomplished the present invention.

That is, the present invention relates to the following items [1] to [4].
[1] An intraoral soluble-type film preparation, comprising a drug layer containing: a drug having a bitter taste and/or an astringent taste, an alkyl sulfate at a content of 3/4 mol or more with respect to 1 mol of the drug and a water-soluble polymer, wherein
   the alkyl sulfate is sodium lauryl sulfate,
   the film preparation comprises coating layers on both sides of the drug layer, and
   a solvent used for formation of the drug layer is an ethanol-containing solvent.
[2] The film preparation according to the item [1], in which the content of the alkyl sulfate is from 3/4 to 3 mol with respect to 1 mol of the drug.
[3] The film preparation according to the item [1] or [2], in which the ethanol-containing solvent used for formation of the drug layer is ethanol or a water-ethanol solution.
[4] The film preparation according to any one of the items [1] to [3], in which the drug is a basic drug.

### Advantageous Effects of Invention

Although the intraoral soluble-type film preparation of the present invention is dissolved rapidly in the oral cavity, the bitter taste and/or astringent taste of a drug having a bitter taste and/or an astringent taste is masked and the film preparation can be administered with good sensation. In addition, the film preparation can be produced without any special apparatus under easy supervision because it requires no special pretreatment step during production.

### Description of Embodiments

The intraoral soluble-type film preparation of the present invention comprises a drug layer containing: (A) a drug having a bitter taste and/or an astringent taste, (B) an alkyl sulfate at a content of 3/4 mol or more with respect to 1 mol of the drug and (C) a water-soluble polymer, wherein
the alkyl sulfate is sodium lauryl sulfate,
the film preparation comprises coating layers on both sides of the drug layer, and
a solvent used for formation of the drug layer is an ethanol-containing solvent.

The drug (A) having a bitter taste and/or an astringent taste to be used in the present invention may be any drug which can be orally administered and has a bitter taste and/or an astringent taste. Specific examples of such drug having a bitter taste and/or an astringent taste include an antipyretic analgesic antiphlogistic such as acetaminophen, isopropylantipyrine, ibuprofen, etodolac, epirizole, ketoprofen, diclofenac sodium, piroxicam and flufenamic acid; a steroidal anti-inflammatory agent such as prednisolone; an antiulcer agent such as ecabet sodium, cimetidine, nizatidine, famotidine, ranitidine hydrochloride, lafutidine, rebamipide and roxatidine acetate hydrochloride; a coronary vasodilator such as dipyridamole and diltiazem hydrochloride; and a peripheral vasodilator such as hydralazine hydrochloride and niceritrol. In addition, an antibiotic such as azithromycin, erythromycin, clarithromycin, chloramphenicol and bacampicillin hydrochloride; a synthetic antibacterial drug such as enoxacin; and an antiviral agent such as acyclovir are included.

In addition, an antispastic such as propantheline bromide, scopolamine hydrobromide and N-methylscopolamine methylsulfate; an antitussive such as dimemorfan phosphate, dextromethorphan hydrobromide and methylephedrine hydrochloride; an expectorant such as ethyl cysteine hydrochloride and bromhexine hydrochloride; and a bronchodilator such as aminophylline, diprophylline and theophylline are included.

In addition, a cardiotonic drug such as caffeine and docarpamine; and a diuretic such as acetazolamide, azosemide, isosorbide and hydrochlorothiazide are included.

In addition a muscle relaxant such as pridinol mesylate and methocarbamol; and a brain metabolic stimulant such as calcium hopantenate and meclofenoxate hydrochloride are included. A minor tranquilizer such as chlordiazepoxide and diazepam; and a major tranquilizer such as chlorpromazine are included.

In addition, a β-blocker such as alprenolol hydrochloride and propranolol hydrochloride are included. An antiarrhythmic agent such as quinidine sulfate is included. A gout remedy such as bucolome and probenecid is included.

In addition, an anticoagulant such as ticlopidine hydrochloride is included. An antimigraine drug such as ergotamine tartrate and anhydrous caffeine; and an antiepileptic such as carbamazepine, topiramate, sodium valproate and lamotrigine are included. An antiallergic agent such as epinastine hydrochloride, olopatadine hydrochloride, chlorpheniramine maleate, ketotifen fumarate, diphenhydramine, cetirizine hydrochloride, bepotastine besilate, mequitazine and loratadine is included. An antiemetic such as trimebutine maleate, betahistine mesilate and mosapride citrate is included. An antihypertensive such as atenolol, amlodipine besilate, alacepril and indapamide is included. A hypolipidemic agent such as simvastatin, pitavastatin calcium and pravastatin sodium is included.

A sympathomimetic agent such as etilefrine hydrochloride is included. An agent for treatment of Alzheimer's dementia such as donepezil hydrochloride is included. An antitumor agent such as capecitabine and tegafur is included- An alkaloidal narcotics such as codeine is included. A vitamin compound such as calciumascorbate, thiamine nitrate, nicotinamide, pyridoxine hydrochloride, fursultiamine hydrochloride and riboflavin is included. A therapeutic agent for pollakiuria such as solifenacin succinate, flavoxate hydrochloride and propiverine hydrochloride is included. An angiotensin converting enzyme inhibitor such as alacepril is included. An erectile dysfunction therapy such as avanafil, sildenafil citrate, tadalafil and vardenafil hydrochloride is included. A therapeutic agent for secondary hyperparathyroidism in maintenance dialysis such as cinacalcet hydrochloride is included. A therapeutic drug for insomnia such as zolpidem tartrate is included. A plasmin antagonist such as tranexamic acid is included. An antidiarrheal such as loperamide hydrochloride is included. A therapeutic drug for threatened abortion and premature delivery such as ritodrine hydrochloride is included. A cholagogue such as ursodeoxycholic acid is included. An antidepressant such as paroxetine hydrochloride is included. An antidiabetic such as pioglitazone hydrochloride and mitiglinide calcium is included. A therapeutic drug for schizophrenia such as risperidone is included.

Of those drugs, basic drugs are preferred from the viewpoint of a masking effect on a bitter taste and/or an astringent taste.

Of those, the following drugs are more preferred: famotidine, olopatadine hydrochloride, chlorpheniramine maleate, ketotifen fumarate, loratadine, amlodipine besylate, donepezil hydrochloride, zolpidem tartrate, loperamide hydrochloride, paroxetine hydrochloride, solifenacin succinate and propiverine hydrochloride. The following drugs are even more preferred: donepezil hydrochloride, paroxetine hydrochloride, solifenacin succinate and propiverine hydrochloride.

The content of the drug may be any content within a range of the amount accepted as a dose of the drug, and usually, the dose is preferably from about 0.001 mg to 50 mg.

In the present invention, the alkyl sulfate (B) has an effect of masking the bitter taste and/or the astringent taste of a drug having a bitter taste and/or an astringent taste. It is important that the content of the alkyl sulfate be 3/4 mol or more with respect to 1 mol of the drug having a bitter taste and/or an astringent taste from the viewpoint of the effect of masking the bitter taste and/or the astringent taste, and the content is preferably from 3/4 to 3 mol, more preferably from 5/6 to 3 mol, even more preferably from 1 to 3 mol, from the viewpoint of the effect of masking the bitter taste and/or the astringent taste and harsh taste. The alkyl sulfate is sodium lauryl sulfate.

Any edible water-soluble polymer may be used as the water-soluble polymer (C) to be used for the drug layer of the present invention. Examples thereof include hydroxypropylcellulose (HPC), hydroxypropylmethylcellulose (HPMC, synonym: hypromellose), hydroxyethylcellulose (HEC), carboxymethylcellulose sodium (CMC-Na, synonym: carmellose sodium), carboxymethylcellulose calcium (CMC-Ca, synonym: carmellose calcium), carboxymethylcellulose potassium (CMC-K, synonym: carmellose potassium), carboxymethylcellulose (CMC, synonym: carmellose), methylcellulose, polyvinylpyrrolidone (PVP), sodium alginate, polyvinyl alcohol (PVA), pullulan, pregelatinized starch and xanthan gum. The polymers may be used singly or in combination. Of those, HPC and HPMC are more preferred from the viewpoint of solubility or disintegration property of the film preparation in the oral cavity. It should be noted that, although the viscosity of each of HPC and HPMC is not particularly limited, for example, HPC has a kinetic viscosity of preferably from 2.0 to 10 mPa·s, more preferably from 3.0 to 10 mPa·s in a 2% aqueous solution at 20°C, and HPMC has a kinetic viscosity of preferably from 3 . 0 to 10 mPa·s, more preferably from 3.0 to 6 mPa·s in a 2% aqueous solution at 20°C. The kinetic viscosity values are based on the test method described in the Japanese Pharmacopoeia 16th Edition.

The content of the water-soluble polymer in the drug layer is preferably from 10 to 98 % by weight, more preferably from 20 to 80 % by weight, even more preferably from 30 to 65 % by weight, from the viewpoint of solubility or disintegration property of the drug.

In addition to the above-mentioned components, the drug layer of the film preparation of the present invention may contain a saccharide, a plasticizer, a sweetener, a coloring agent, or a flavor as well as a solvent such as water or ethanol. Examples of the saccharide include maltose, reduced maltose starch syrup, maltitol, erythritol, xylitol, sucrose, sorbitol, mannitol and trehalose. Examples of the plasticizer include polyethylene glycol, glycerin, sorbitol and triethyl citrate. Examples of the sweetener include sodium saccharin, aspartame, acesulfame potassium and sucralose. Examples of the coloring agent include titanium oxide, red ferric oxide, yellow ferric oxide and an edible dye.

Usually, the film preparation of the present invention is preferably a three-layer film preparation having coating layers on both sides of the drug layer. It should be noted that the drug layer may contain two layers . Even when water is used as a solvent used for formation of the drug layer in the film preparation of the present invention, the effect of masking the bitter taste and/or the astringent taste can be achieved. However, an ethanol-containing solvent is used from the viewpoints of uniform blending of the drug in the drug layer and masking of the bitter taste and/or the astringent taste. The ethanol-containing solvent is preferably ethanol or a water-ethanol solution, and the water-ethanol solution contains ethanol at a concentration of preferably from 20 to 100 % by weight, more preferably from 40 to 100 % by weight.

A production method for the film preparation of the present invention, for example, a production method for a film preparation having coating layers on both sides of the drug layer includes: forming coating layers; forming drug layers on the coating layers; and sticking and laminating the two thus-obtained layers so that the drug layers are present inside. That is, the coating layer may be formed by preparing a coating layer solution and spreading the solution on a release film, followed by drying. Next, the drug layer may be formed by spreading a drug layer solution on the coating layers, followed by drying. The resultant laminate composed of the drug layer and coating layer may be thermally compressed together with another laminate using a laminating machine so that the drug layers are faced to each other, to thereby produce a film preparation having the coating layers on both sides of the drug layers. The drug layers of the film preparation produced in this production method and obtained by sticking and laminating the drug layers having the same composition are defined as a single layer.

In addition, the film preparation may be produced by a method including forming a coating layer by spreading and drying a coating layer solution on the drug layer of the laminate composed of the drug layer and coating layer.

The drug layer solution can be obtained by dissolving or dispersing components necessary for the drug layer in an ethanol-containing solvent. In this procedure, the total concentration of the drug, the alkyl sulfate and the water-soluble polymer is preferably from 10 to 80 % by weight, more preferably from 20 to 70 % by weight, even more preferably from 25 to 60 % by weight, from the viewpoints of masking of the bitter taste and/or the astringent taste, sensation upon administration, dispersibility of the drug, shortening of drying time and spreadability.

The coating layer may be any coating layer included in a general film preparation, and contains a water-soluble polymer, a saccharide, a plasticizer, a sweetener, a coloring agent, a flavor, etc. Examples of the water-soluble polymer, saccharide, plasticizer, sweetener, coloring agent and flavor to be used in the coating layer include the ones listed as components of the drug layer. The coating layer contains the water-soluble polymer at a content of preferably from 20 to 98 % by weight, more preferably from 30 to 90 % by weight, even more preferably from 40 to 80 % by weight. The coating layer solution can be prepared in the same manner as the drug layer solution and the coating layer can be formed by the same means as the drug layer. Water, ethanol, or a water-ethanol solution may be used as the solvent.

The film preparation of the present invention may have a multilayer structure including three or more layers, and the thickness of the film preparation is preferably from 10 to 1,000 µm, more preferably from 20 to 500 µm, even more preferably from 30 to 300 µm. When the thickness of the preparation is too large, the preparation is dissolved or disintegrated slowly in the oral cavity and bad sensation is felt when the preparation is administered. On the other hand, when the thickness of the preparation is too small, handling property of the preparation is deteriorated, or it becomes difficult to incorporate a required amount of the drug. It should be noted that the size of the film preparation is not particularly limited as long as the film preparation can be administered easily, and for example, the size is preferably from about 1 to 7 cm². In addition, the shape of the film preparation is not particularly limited as long as the film preparation can be administered easily, and for example, the shape may be appropriately selected from a circle, an ellipse, and a square.

The film preparation of the present invention can achieve rapid dissolution or disintegration of the drug and is excellent in solubility in water. For example, the time for dissolution or disintegration in the oral cavity is preferably 1 minute or less, more preferably 30 seconds or less, while the time for dissolution in water is preferably 3 minutes or less, more preferably 1 minute or less.

In addition, the film preparation of the present invention can have a sheet form in which a plurality of preparations are arranged in rows and columns and cutting lines for demarcation of the shapes of the preparations are provided. A resin film may be provided on one side thereof . Such preparations can be separated along with the cut lines, peeled off from the resin film, and taken one by one by a patient. A film appropriately selected from films formed of the following resins may be used as such resin film: polyethylene terephthalate, polyethylene naphthalate, copolyester, polyimide, polypropylene, cellulose triacetate, a vinyl acetate resin, an ethylene-vinyl acetate copolymer, polyethylene, polyvinyl chloride, polycarbonate, polypropylene, triacetate, and fluororesin (ETFE, PFA, FEP). Of those resins, polyethylene terephthalate (PET) is more preferred.

### Examples

Next, the present invention is described in detail by way of Examples. Preparation Examples 1-3, 6-8, 17-19, 21-23, and 25-27 are reference examples.

### Test Example 1

### [Preparation Example 1]

11.38 parts by weight of paroxetine hydrochloride hydrate (10 parts by weight in terms of paroxetine) and 15 parts by weight of hydroxypropylcellulose (HPC, NIPPON SODA CO., LTD.) were added to 40 parts by weight of purified water, and the mixture was mixed by stirring, thereby obtaining a drug layer solution.

In addition, 15 parts by weight of hypromellose (Shin-Etsu Chemical Co., Ltd.), 2 parts by weight of triethyl citrate, 2 parts by weight of maltitol, 0.5 part by weight of sucralose and 1 part by weight of titanium oxide were added to a mixed solvent of purified water/ethanol (30 parts by weight/30 parts by weight), and the mixture was mixed by stirring, thereby obtaining a coating layer solution.

Firstly, the coating layer solution was spread on a polyester release film and dried to form a coating layer, and then the drug layer solution was spread on the coating layer and dried to form a drug layer on the coating layer, thereby obtaining a two-layer structure film.

Two sets of the two-layer structure films were prepared and thermally compressed using a laminating machine so that the drug layers of the two sets of the two-layer structure films were faced to each other, thereby obtaining a three-layer structure film composed of the coating layer/drug layer/coating layer. The three-layer structure film was cut into a size of 24x18 mm, thereby obtaining a film preparation.

### [Preparation Example 2]

A film preparation was obtained in the same manner as in Preparation Example 1 except that 40 parts by weight of ethanol was used instead of 40 parts by weight of purified water in the drug layer solution used in Preparation Example 1.

### [Preparation Example 3]

A film preparation was obtained in the same manner as in Preparation Example 1 except that 9 parts by weight of sodium lauryl sulfate was further added to the drug layer solution used in Preparation Example 1.

### [Preparation Example 4]

A film preparation was obtained in the same manner as in Preparation Example 3 except that a mixed solvent of purified water/ethanol (20 parts by weight/20 parts by weight) was used instead of 40 parts by weight of purified water in the drug layer solution used in Preparation Example 3.

### [Preparation Example 5]

A film preparation was obtained in the same manner as in Preparation Example 3 except that 40 parts by weight of ethanol was used instead of 40 parts by weight of purified water in the drug layer solution used in Preparation Example 3.

The film preparations obtained in Preparation Examples 1 to 5 were each evaluated for its bitter taste and astringent taste by three panelists (described as 1 to 3 in tables) . That is, each of the preparations was dissolved in the mouth and evaluated based on the following criteria. Table 1 shows the evaluation results.

### Evaluation criteria

1: A bitter taste/astringency is unnoticeable or virtually unnoticeable.
2: A bitter taste/astringency is slightly noticeable.
3: A bitter taste/astringency is noticeable but acceptable.
4: A bitter taste/astringency is strong and unacceptable.
5: A bitter taste/astringency is very strong and leads to suffering.

**[Table 1]**

| Formulation | | Preparation Example 1 | Preparation Example 2 | Preparation Example 3 | Preparation Example 4 | Preparation Example 5 |
|---|---|---|---|---|---|---|
| Coating layer | Hypromellose | 15 | 15 | 15 | 15 | 15 |
| | Triethyl citrate | 2 | 2 | 2 | 2 | 2 |
| | Maltitol | 2 | 2 | 2 | 2 | 2 |
| | Sucralose | 0.5 | 0.5 | 0.5 | 0.5 | 0.5 |
| | Titanium oxide | 1 | 1 | 1 | 1 | 1 |
| | Purified water | 30 | 30 | 30 | 30 | 30 |
| | Ethanol | 30 | 30 | 30 | 30 | 30 |
| Drug layer | Paroxetine hydrochloride hydrate | 11.38 | 11.38 | 11.38 | 11.38 | 11.38 |
| | HPC | 15 | 15 | 15 | 15 | 15 |
| | Sodium lauryl sulfate | - | - | 9 | 9 | 9 |
| | Purified water | 40 | - | 40 | 20 | - |
| | Ethanol | - | 40 | - | 20 | 40 |
| Bitter taste | 1 | 5 | 5 | 3 | 2 | 1 |
| | 2 | 4 | 4 | 3 | 3 | 3 |
| | 3 | 4 | 5 | 3 | 3 | 2 |
| Astringency | 1 | 5 | 5 | 3 | 2 | 1 |
| | 2 | 5 | 5 | 3 | 3 | 2 |
| | 3 | 5 | 5 | 3 | 3 | 2 |

### Preparation examples 1-3 are not in the scope of claims.

Table 1 shows that, when the film preparations obtained by blending the alkyl sulfate in the drug layer containing the drug having a bitter taste and/or an astringent taste contain the alkyl sulfate at a content of 3/4 mol or more with respect to 1 mol of the drug in the drug layer, the bitter taste and/or the astringent taste is masked. The table further shows that, when the solvent used in production of the drug layer contains ethanol, the bitter taste and astringent taste of the drug blended can be significantly masked (Preparation Examples 4 and 5).

### Test Example 2

### [Preparation Example 6]

A film preparation was obtained in the same manner as in Preparation Example 2 except that 9 parts by weight of sodium lauryl sulfate were further added to the coating layer solution used in Preparation Example 2.

The film preparations obtained in Preparation Examples 2, 5 and 6 were each evaluated for its bitter taste and astringent taste simultaneously with Test Example 1. Table 2 shows the results .

**[Table 2]**

| Formulation | | Preparation Example 2 | Preparation Example 5 | Preparation Example 6 |
|---|---|---|---|---|
| Coating layer | Hypromellose | 15 | 15 | 15 |
| | Triethyl citrate | 2 | 2 | 2 |
| | Maltitol | 2 | 2 | 2 |
| | Sucralose | 0.5 | 0.5 | 0.5 |
| | Titanium oxide | 1 | 1 | 1 |
| | Sodium lauryl sulfate | - | - | 9 |
| | Purified water | 30 | 30 | 30 |
| | Ethanol | 30 | 30 | 30 |
| Drug layer | Paroxetine hydrochloride hydrate | 11.38 | 11.38 | 11.38 |
| | HPC | 15 | 15 | 15 |
| | Sodium lauryl sulfate | - | 9 | - |
| | Ethanol | 40 | 40 | 40 |
| Bitter taste | 1 | 5 | 1 | 4 |
| | 2 | 4 | 3 | 3 |
| | 3 | 5 | 2 | 4 |
| Astringency | 1 | 5 | 1 | 3 |
| | 2 | 5 | 2 | 2 |
| | 3 | 5 | 2 | 4 |

### Preparation examples 2 and 6 are not in the scope of claims.

Table 2 shows that the alkyl sulfate has no effect masking the bitter taste and the astringent taste even when added to the coating layer and should be blended in the drug layer to provide an interaction with the drug having the bitter taste and/or the astringent taste.

### Test Example 3

### [Preparation Example 7] to [Preparation Example 12]

Film preparations were obtained in the same manner as in Preparation Example 5 except that the amount of sodium lauryl sulfate in the drug layer solution used in Preparation Example 5 was changed from 9 parts by weight to 2.25, 4.5, 6.75, 13.5, 18, and 27 parts by weight, respectively.

The film preparations obtained in Preparation Examples 2, 5 and 7 to 12 were each evaluated for its bitter taste and astringent taste in the same manner as in Test Example 1. Table 3 shows the results. Preparation examples 2, 7, 8 are not in the scope of claims.

**[Table 3]**

| Formulation | | Preparation Example 2 | Preparation Example 7 | Preparation Example 8 | Preparation Example 9 | Preparation Example 5 | Preparation Example 10 | Preparation Example 11 | Preparation Example 12 |
|---|---|---|---|---|---|---|---|---|---|
| Coating layer | Hypromellose | 15 | 15 | 15 | 15 | 15 | 15 | 15 | 15 |
| | Triethyl citrate | 2 | 2 | 2 | 2 | 2 | 2 | 2 | 2 |
| | Maltitol | 2 | 2 | 2 | 2 | 2 | 2 | 2 | 2 |
| | Sucralose | 0.5 | 0.5 | 0.5 | 0.5 | 0.5 | 0.5 | 0.5 | 0.5 |
| | Titanium oxide | 1 | 1 | 1 | 1 | 1 | 1 | 1 | 1 |
| | Purified water | 30 | 30 | 30 | 30 | 30 | 30 | 30 | 30 |
| | Ethanol | 30 | 30 | 30 | 30 | 30 | 30 | 30 | 30 |
| Drug layer | Paroxetine hydrochlorid e hydrate | 11.38 | 11.38 | 11.38 | 11.38 | 11.38 | 11.38 | 11.38 | 11.38 |
| | HPC | 15 | 15 | 15 | 15 | 15 | 15 | 15 | 15 |
| | Sodium lauryl sulfate (SLS) | - | 2.25 | 4.5 | 6.75 | 9 | 13.5 | 18 | 27 |
| | Ethanol | 40 | 40 | 40 | 40 | 40 | 40 | 40 | 40 |
| | Ratio of SLS (with respect to mol of the drug) | 0 | 1/4 | 1/2 | 3/4 | 1 | 3/2 | 2 | 3 |
| Bitter taste | 1 | 5 | 5 | 4 | 2 | 1 | 1 | 1 | 2 |
| | 2 | 4 | 4 | 4 | 3 | 3 | 3 | 2 | 3 |
| | 3 | 5 | 4 | 4 | 3 | 2 | 2 | 2 | 3 |
| Astringency | 1 | 5 | 5 | 4 | 3 | 1 | 1 | 1 | 1 |
| | 2 | 5 | 5 | 3 | 2 | 2 | 2 | 2 | 2 |
| | 3 | 5 | 5 | 3 | 3 | 2 | 2 | 2 | 2 |

Table 3 shows that an excellent masking effect can be achieved when the content of the alkyl sulfate in the drug layer is 3/4 mol or more with respect to 1 mol of the drug having the bitter taste and/or the astringent taste. It should be noted that when the content of the alkyl sulfate exceeded 3 mol with respect to 1 mol of the drug, harsh taste originating from the alkyl sulfate was detected.

### Test Example 4

### [Preparation Example 13] to [Preparation Example 16]

Film preparations were obtained in the same manner as in Preparation Example 5 except that the amount of ethanol in the drug layer solution used in Preparation Example 5 was changed from 40 parts by weight to 20, 30, 50 and 60 parts by weight, respectively.

The film preparations obtained in Preparation Examples 5 and 13 to 16 were each evaluated for its bitter taste and astringent taste in the same manner as in Test Example 1. Table 4 shows the results.

**[Table 4]**

| Formulation | | Preparation Example 13 | Preparation Example 14 | Preparation Example 5 | Preparation Example 15 | Preparation Example 16 |
|---|---|---|---|---|---|---|
| Coating layer | Hypromellose | 15 | 15 | 15 | 15 | 15 |
| | Triethyl citrate | 2 | 2 | 2 | 2 | 2 |
| | Maltitol | 2 | 2 | 2 | 2 | 2 |
| | Sucralose | 0.5 | 0.5 | 0.5 | 0.5 | 0.5 |
| | Titanium oxide | 1 | 1 | 1 | 1 | 1 |
| | Purified water | 30 | 30 | 30 | 30 | 30 |
| | Ethanol | 30 | 30 | 30 | 30 | 30 |
| Drug layer | Paroxetine hydrochloride hydrate | 11.38 | 11.38 | 11.38 | 11.38 | 11.38 |
| | HPC | 15 | 15 | 15 | 15 | 15 |
| | Sodium lauryl sulfate | 9 | 9 | 9 | 9 | 9 |
| | Ethanol | 20 | 30 | 40 | 50 | 60 |
| Bitter taste | 1 | 1 | 1 | 1 | 1 | 1 |
| | 2 | 3 | 3 | 3 | 3 | 3 |
| | 3 | 2 | 2 | 2 | 2 | 2 |
| Astringency | 1 | 1 | 1 | 1 | 2 | 2 |
| | 2 | 2 | 2 | 2 | 2 | 2 |
| | 3 | 1 | 2 | 2 | 2 | 2 |

Table 4 shows that an excellent effect of masking the bitter taste and the astringent taste can be achieved by using the ethanol-containing solvent as a solvent used in production of the drug layer. In addition, the amount of ethanol used was found not to affect the masking effect.

### Test Example 5

### [Preparation Example 17]

A film preparation was obtained in the same manner as in Preparation Example 1 except that 5 parts by weight of donepezil hydrochloride was used instead of 11.38 parts by weight of paroxetine hydrochloride hydrate in the drug layer solution used in Preparation Example 1.

### [Preparation Example 18]

A film preparation was obtained in the same manner as in Preparation Example 17 except that 40 parts by weight of ethanol was used instead of 40 parts by weight of purified water in the drug layer solution used in Preparation Example 17.

### [Preparation Example 19]

A film preparation was obtained in the same manner as in Preparation Example 17 except that 3.5 parts by weight of sodium lauryl sulfate was further added to the drug layer solution used in Preparation Example 17.

### [Preparation Example 20]

A film preparation was obtained in the same manner as in Preparation Example 19 except that 40 parts by weight of ethanol was used instead of 40 parts by weight of purified water in the drug layer solution used in Preparation Example 19.

The film preparations obtained in Preparation Examples 17 to 20 were each evaluated for its bitter taste and astringent taste in the same manner as in Test Example 1. Table 5 shows the results.

**[Table 5]**

| Formulation | | Preparation Example 17 | Preparation Example 18 | Preparation Example 19 | Preparation Example 20 |
|---|---|---|---|---|---|
| Coating layer | Hypromellose | 15 | 15 | 15 | 15 |
| | Triethyl citrate | 2 | 2 | 2 | 2 |
| | Maltitol | 2 | 2 | 2 | 2 |
| | Sucralose | 0.5 | 0.5 | 0.5 | 0.5 |
| | Titanium oxide | 1 | 1 | 1 | 1 |
| | Purified water | 30 | 30 | 30 | 30 |
| | Ethanol | 30 | 30 | 30 | 30 |
| Drug layer | Donepezil hydrochloride | 5 | 5 | 5 | 5 |
| | HPC | 15 | 15 | 15 | 15 |
| | Sodium lauryl sulfate | - | - | 3.5 | 3.5 |
| | Purified water | 40 | - | 40 | - |
| | Ethanol | - | 40 | - | 40 |
| Bitter taste | 1 | 4 | 3 | 1 | 1 |
| | 2 | 4 | 4 | 3 | 2 |
| | 3 | 3 | 3 | 1 | 1 |
| Astringency | 1 | 4 | 3 | 2 | 1 |
| | 2 | 3 | 3 | 2 | 1 |
| | 3 | 4 | 4 | 1 | 1 |

### Preparation examples 17-19 are not in the scope of claims.

Table 5 shows that, also when the drug layers of the film preparations containing donepezil hydrochloride instead of paroxetine hydrochloride contain the alkyl sulfate at a content of 3/4 mol or more with respect to 1 mol of the drug, the bitter taste and/or the astringent taste can be masked. The table further shows that the bitter taste and/or the astringent taste can be masked by using the ethanol-containing solvent as the solvent used for formation of the drug layer.

### Test Example 6

### [Preparation Example 21]

A film preparation was obtained in the same manner as in Preparation Example 1 except that 10 parts by weight of propiverine hydrochloride was used instead of 11.38 parts by weight of paroxetine hydrochloride hydrate in the drug layer solution used in Preparation Example 1.

### [Preparation Example 22]

A film preparation was obtained in the same manner as in Preparation Example 21 except that 40 parts by weight of ethanol was used instead of 40 parts by weight of purified water in the drug layer solution used in Preparation Example 21.

### [Preparation Example 23]

A film preparation was obtained in the same manner as in Preparation Example 21 except that 7.5 parts by weight of sodium lauryl sulfate was further added to the drug layer solution used in Preparation Example 21 and the amount of purified water in the drug layer solution was changed from 40 parts by weight to 80 parts by weight.

### [Preparation Example 24]

A film preparation was obtained in the same manner as in Preparation Example 23 except that 40 parts by weight of ethanol was used instead of 80 parts by weight of purified water in the drug layer solution used in Preparation Example 23.

The film preparations obtained in Preparation Examples 21 to 24 were each evaluated for its bitter taste and astringent taste in the same manner as in Test Example 1. Table 6 shows the results.

**[Table 6]**

| Formulation | | Preparation Example 21 | Preparation Example 22 | Preparation Example 23 | Preparation Example 24 |
|---|---|---|---|---|---|
| Coating layer | Hypromellose | 15 | 15 | 15 | 15 |
| | Triethyl citrate | 2 | 2 | 2 | 2 |
| | Maltitol | 2 | 2 | 2 | 2 |
| | Sucralose | 0.5 | 0.5 | 0.5 | 0.5 |
| | Titanium oxide | 1 | 1 | 1 | 1 |
| | Purified water | 30 | 30 | 30 | 30 |
| | Ethanol | 30 | 30 | 30 | 30 |
| Drug layer | Propiverine hydrochloride | 10 | 10 | 10 | 10 |
| | HPC | 15 | 15 | 15 | 15 |
| | Sodium lauryl sulfate | - | - | 7.5 | 7.5 |
| | Purified water | 40 | - | 80 | - |
| | Ethanol | - | 40 | - | 40 |
| Bitter taste | 1 | 4 | 5 | 1 | 1 |
| | 2 | 3 | 2 | 1 | 2 |
| | 3 | 3 | 3 | 1 | 1 |
| Astringency | 1 | 5 | 5 | 1 | 1 |
| | 2 | 5 | 4 | 1 | 1 |
| | 3 | 5 | 5 | 2 | 1 |

### Preparation examples 21-23 are not in the scope of claims.

Table 6 shows that, also when the drug layers of the film preparations containing propiverine hydrochloride instead of paroxetine hydrochloride contain the alkyl sulfate at a content of 3/4 mol or more with respect to 1 mol of the drug, the bitter taste and/or the astringent taste can be masked. The effect of suppressing of bitter taste and/or astringent taste of propiverine hydrochloride was found to be significant as is the case of paroxetine hydrochloride hydrate and donepezil hydrochloride. On the other hand, the effect provided when ethanol was used was considered to be larger although there was a relatively small difference depending on the kind of the solvent.

### Test Example 7

### [Preparation Example 25]

A film preparation was obtained in the same manner as in Preparation Example 1 except that 2.5 parts by weight of solifenacin succinate was used instead of 11.38 parts by weight of paroxetine hydrochloride hydrate in the drug layer solution used in Preparation Example 1.

### [Preparation Example 26]

A film preparation was obtained in the same manner as in Preparation Example 25 except that 40 parts by weight of ethanol was used instead of 40 parts by weight of purified water in the drug layer solution used in Preparation Example 25.

### [Preparation Example 27]

A film preparation was obtained in the same manner as in Preparation Example 25 except that 1.5 parts by weight of sodium lauryl sulfate was further added to the drug layer solution used in Preparation Example 25.

### [Preparation Example 28]

A film preparation was obtained in the same manner as in Preparation Example 27 except that 40 parts by weight of ethanol was used instead of 40 parts by weight of purified water in the drug layer solution used in Preparation Example 27.

The film preparations obtained in Preparation Examples 25 to 28 were each evaluated for its bitter taste and astringent taste in the same manner as in Test Example 1. Table 7 shows the results.

**[Table 7]**

| Formulation | | Preparation Example 25 | Preparation Example 26 | Preparation Example 27 | Preparation Example 28 |
|---|---|---|---|---|---|
| Coating layer | Hypromellose | 15 | 15 | 15 | 15 |
| | Triethyl citrate | 2 | 2 | 2 | 2 |
| | Maltitol | 2 | 2 | 2 | 2 |
| | Sucralose | 0.5 | 0.5 | 0.5 | 0.5 |
| | Titanium oxide | 1 | 1 | 1 | 1 |
| | Purified water | 30 | 30 | 30 | 30 |
| | Ethanol | 30 | 30 | 30 | 30 |
| Drug layer | Solifenacin succinate | 2.5 | 2.5 | 2.5 | 2.5 |
| | HPC | 15 | 15 | 15 | 15 |
| | Sodium lauryl sulfate | - | - | 1.5 | 1.5 |
| | Purified water | 40 | - | 40 | - |
| | Ethanol | - | 40 | - | 40 |
| Bitter taste | 1 | 3 | 3 | 1 | 1 |
| | 2 | 2 | 2 | 2 | 2 |
| | 3 | 3 | 2 | 1 | 1 |
| Astringency | 1 | 4 | 4 | 2 | 1 |
| | 2 | 3 | 3 | 1 | 1 |
| | 3 | 4 | 3 | 1 | 2 |

### Preparation examples 25-27 are not in the scope of claims.

Table 7 shows that, also when the drug layers of the film preparations containing solifenacin succinate instead of paroxetine hydrochloride contain the alkyl sulfate at a content of 3/4 mol or more with respect to 1 mol of the drug, the bitter taste and/or the astringent taste can be masked. The effect of suppressing the bitter taste and/or astringent taste of solifenacin succinate was found to be significant as is the case of paroxetine hydrochloride hydrate, donepezil hydrochloride and propiverine hydrochloride. On the other hand, the effect provided when ethanol was used was considered to be larger although there was a relatively small difference depending on the kind of the solvent.

### Test Example 8

The states of the drug layer solutions used in production of Preparation Examples were visually observed at the time of preparation of the solutions and the next day (24 hours) of preparation. As a result, in Preparation Examples 1, 2, 7, 8, 18, 21 and 22 showing no effect of masking the bitter taste and/or the astringent taste, precipitates of the drug were found in the solution the next day, and in Preparation Examples 17, 25 and 26, the drugs were dissolved and clear solutions were obtained. On the other hand, in Preparation Examples 4, 5, 9 to 16, 19, 20, 23, 24, 27 and 28 showing the effect of masking the bitter taste and/or the astringent taste, no precipitate was found in the solutions even the next day and to the solutions contained the drug in a uniform dispersion state. This is probably because the drug and the alkyl sulfate formed an ion pair. In Preparation Example 6 in which the alkyl sulfate was added to the coating layer, almost no effect of masking the bitter taste and/or the astringent taste was observed. That is, the effect of masking the bitter taste and/or the astringent taste of the present invention is considered to be provided by formation of the ion pair of the drug and the alkyl sulfate.

## Claims

1. An intraoral soluble-type film preparation, comprising a drug layer containing: a drug having a bitter taste and/or an astringent taste; an alkyl sulfate at a content of 3/4 mol or more with respect to 1 mol of the drug; and a water-soluble polymer,
wherein
the alkyl sulfate is sodium lauryl sulfate,
the film preparation comprises coating layers on both sides of the drug layer, and
a solvent used for formation of the drug layer is an ethanol-containing solvent.

2. The film preparation according to claim 1, wherein the content of the alkyl sulfate is from 3/4 to 3 mol with respect to 1 mol of the drug.

3. The film preparation according to claim 1 or 2, wherein the ethanol-containing solvent used for formation of the drug layer is ethanol or a water-ethanol solution.

4. The film preparation according to any one of claims 1 to 3, wherein the drug is a basic drug.

## Patentansprüche

1. Filmzubereitung von intraoral löslicher Art, umfassend eine Wirkstoffschicht, enthaltend:
einen Wirkstoff mit bitterem Geschmack und/oder adstringierendem Geschmack; ein Alkylsulfat mit einem Gehalt von einem ¾ Mol oder mehr in Bezug auf 1 Mol des Wirkstoffs; und ein wasserlösliches Polymer,
wobei
das Alkylsulfat Natriumlaurylsulfat ist,
die Filmzubereitung Beschichtungslagen auf beiden Seiten der Wirkstoffschicht umfasst und wobei das zum Ausbilden der Wirkstoffschicht verwendete Lösungsmittel ein Ethanol-haltiges Lösungsmittel ist.

2. Filmzubereitung nach Anspruch 1, wobei der Gehalt des Alkylsulfats von einem ¾ Mol bis 3 Mol in Bezug of 1 Mol des Wirkstoffs beträgt.

3. Filmzubereitung nach Anspruch 1 oder 2, wobei das zum Ausbilden der Wirkstoffschicht verwendete Ethanol-haltige Lösungsmittel Ethanol oder eine Wasser-Ethanol-Lösung ist.

4. Filmzubereitung nach einem der Ansprüche 1 bis 3, wobei der Wirkstoff ein Basiswirkstoff ist.

## Revendications

1. Préparation de film du type soluble par voie intraorale, comprenant une couche de médicament contenant : un médicament présentant un goût amer et/ou un goût astringent ; un alkylsulfate en une quantité de 3/4 mole ou plus pour 1 mole du médicament ; et un polymère soluble dans l'eau,
dans laquelle
l'alkylsulfate est le laurylsulfate de sodium,
la préparation de film comprend des couches d'enrobage sur les deux côtés de la couche de médicament, et
un solvant utilisé pour la formation de la couche de médicament est un solvant contenant de l'éthanol.

2. Préparation de film selon la revendication 1, dans laquelle la teneur en l'alkylsulfate est de 3/4 à 3 moles pour 1 mole du médicament.

3. Préparation de film selon la revendication 1 ou 2, dans laquelle le solvant contenant de l'éthanol utilisé pour la formation de la couche de médicament est l'éthanol ou une solution aqueuse d'éthanol.

4. Préparation de film selon l'une quelconque des revendications 1 à 3, dans laquelle le médicament est un médicament basique.
